# EUROPEAN PATENT SPECIFICATION

(11) **EP 0 706 984 B1**
(45) Date of publication and mention of the grant of the patent: **02.06.1999**
(21) Application number: 95306975.4
(22) Date of filing: 02.10.1995
(51) Int. Cl.: C07C 5/27, B01J 29/70

(54) **Skeletal isomerization of olefins**
Skelettisomerisierung von Alkenen
Isomérisation squelettale d'oléfines

(30) Priority: 12.10.1994 GB 9420528
(43) Date of publication of application: 17.04.1996
(73) Proprietor: BP Chemicals Limited, London EC2M 7BA (GB)
(72) Inventor: Atkins, Martin Philip, BP Chemicals Limited, Middlesex, TW16 7LN (GB); Smith, Warren John, BP Chemicals Limited, Middlesex, TW16 7LN (GB)
(74) Representative: Krishnan, Suryanarayana Kalyana

(56) References cited:
- EP-A- 0 353 915
- EP-A- 0 523 838
- EP-A- 0 574 994
- WO-A-93/23353
- WO-A-94/08920

## Description

This invention relates to a process for the skeletal isomerization of unsaturated hydrocarbons, especially olefins, using crystalline silicates, germanates, metallosilicates and metallogermanates.

Crystalline (metallo)silicates and (metallo)germanates are well known as are the methods of preparation thereof Crystalline materials of this type which are of particular interest in this invention are zeolites having the general empirical formula:

*m*(M_{2/}_{*a*}O):X₂O₃:*y*YO₂ (I)

in which *m* is 0.5 to 1.5; M is a cation of valency *a*; X is a metal of valency 3 selected from aluminium, boron, gallium and iron; Y is silicon or germanium and *y* is at least 5; and has in its calcined hydrogen form, an x-ray diffraction pattern including significant peaks substantially as shown in Table A herein.

Such materials can be regarded as a metallosilicate, a metallogermanate, or, as a silicate or a germanate when the value of y is very high. These materials are referred to as SUZ-4 zeolites and are claimed and described in our published EP-A-0353915 and will be so referred to here and throughout the specification. This publication refers to a variety of potential applications for SUZ-4 zeolites, particularly as a catalyst or absorbent. It states that this type of material may be used in a number of purifications and separations, "and a number of catalytic conversions, for example the conversion of hydrocarbons and oxygenates into other products.". There is no further elaboration on the so called conversion of hydrocarbons into other products.

It has now been found that the SUZ-4 zeolites are excellent catalysts for skeletal isomerisation of unsaturated hydrocarbons.

Accordingly the present invention is a process for the skeletal isomerization of hydrocarbon feedstock comprising linear olefins to a product enriched in branched olefins in the presence of a crystalline zeolite, characterised in that the crystalline zeolite is a zeolite SUZ-4 of the empirical formula:

*m*(M_{2/}_{*a*}O):X₂O₃:*y*YO₂ (I)

in which *m* is 0.5 to 1.5; M is a cation of valency *a;* X is a metal of valency 3 selected from aluminium, boron, gallium and iron; Y is silicon or germanium and *y* is at least 5; and has in its calcined hydrogen form, an x-ray diffraction pattern including significant peaks substantially as shown in Table A herein.

The hydrocarbon feedstock subjected to skeletal isomerization suitably comprises linear olefins having 4-10 carbon atoms, preferably from 4 to 5 carbon atoms. The source of such feedstock may be the by-products from refinery processes such as steam cracking, fluid catalytic cracking, catalytic cracking and butadiene raffinates (Raffinate I) which contain significant proportions of C4 olefins, or, the unpolymerised by-products recoverable when Raffinate I is polymerised using eg a Lewis acid catalyst to form polybutenes, or, the by-product gases resulting from the production of the lead-free anti-knock compound, methyl tertiary butyl ether. The so-called "Raffinate II" typically contains from 30-55% w/w of butene-1, about 10% w/w of cis-butene-2, about 17% w/w of *trans*-butene-2, up to 6% w/w of iso-butene and up to 30% w/w of the saturated C4 hydrocarbons n-butane and iso-butane. The feedstock may also be derived from commercial products such as the effluent from the conventional process for producing methyl tertiary butyl ether; effluents from a polybutene process; and effluents from a tetraalkyl methyl ether. Thus, such feedstock would suitably comprise either C4 olefin optionally diluted with C3-C12 alkanes, heavy ends and an inert gas such as eg nitrogen. The hydrocarbon feedstock may preferably contain 30-90% w/w and more preferably from 50-70% w/w of the linear olefin. It would be clear from the above that such feedstock may contain moderate amounts of paraffinic hydrocarbons. It should be possible to recycle unconverted linear olefins in the products from the isomerization step back to the isomerization stage after separation of the branched olefins so formed. The concentration of the various components in the hydrocarbon feedstock being recycled can be monitored by removing bleed streams in the process cycle and periodically analysing the same so that the proportion of the various components may be adjusted as desired.

The framework structure of the crystalline, zeolite SUZ-4 in its calcined, hydrogen can be characterised by the XRD pattern thereof shown in Table A below. This is particularly typical of the zeolite in which the atom X is aluminium and the atom Y is silicon, ie the zeolite is an aluminosilicate. In the Tables, the following notations have been used:
- d(Å) -: d-spacing
- I/I_{O} -: Relative Intensity of the peak
- VS -: 60-140
- S -: 40-60
- M -: 20-40
- W -: 0-20

**TABLE A**

| d(Å) | I/I_{O} | d(Å) | I/I_{O} | d(Å) | I/I_{O} |
|---|---|---|---|---|---|
| 11.5 ± 0.25 | VS | 4.00 ± 0.10 | W | 3.49 ± 0.07 | S |
| 7.50 ± 0.15 | M | 3.95 ± 0.08 | M | 3.48 ± 0.07 | M |
| 7.20 ± 0.15 | W | 3.81 ± 0.08 | M | 3.14 ± 0.07 | M |
| 5.88 ± 0.12 | S | 3.75 ± 0.08 | W | 2.97 ± 0.06 | W |
| 5.73 ± 0.12 | M | 3.67 ± 0.08 | W | 2.93 ± 0.06 | M |
| 4.75 ± 0.10 | M | 3.58 ± 0.08 | S | 2.91 ± 0.06 | W |
| 4.58 ± 0.10 | M/S | 3.55 ± 0.08 | S | - | - |

The preparation and characterization of these SUZ-4 zeolites are described extensively in our published EP-A-0353915 and is incorporated herein by reference. In SUZ-4, as in most other zeolites, the concentration of the acid sites in the framework of the hydrogen-form of the zeolite structure is governed by the concentration of the atom X in the framework.

A feature of the present invention is that the skeletal isomerization of the linear olefins in a given hydrocarbon feedstock to the corresponding branched olefins can also be achieved by using a demetallated the hydrogen form of the SUZ-4 zeolite. Demetallation means to reducing the concentration of the X atoms forming the framework of the zeolite structure in its hydrogen-form (the so called "H-Form"), thereby reducing the acidity thereof so that the catalytic activity of the resultant demetallated zeolite can be harnessed for the skeletal isomerization.

The SUZ-4 zeolite in its H-Form may also subjected to demetallation by a variety of methods which enable the ratio of YO₂/X₂O₃ in the framework thereof to be increased. These methods include: the direct synthetic route in which a portion of the X atoms can be replaced eg with a less acidic atom such as eg boron; by a post-synthetic treatment such as eg steaming, acid treatment; or by reaction with a compound of Y such as eg in the case of silicon, SiCl₄. The specific techniques used are well known in the art and are described, for instance, in J Scherzer's "Catalytic Materials Relationship Between Structure and Reactivity", Eds. T E Whyte et al, ACS Symposium Series No. 248, pp 157 (1984).

Where H-SUZ-4 is subjected to such a demetallation technique, it should be noted that a simple and superficial analysis of the demetallated zeolite may indicate that there is no significant change in the relative ratio of its X₂O₃ : YO₂ when compared with the original unmodified zeolite. This is primarily due to the fact that during modification of the zeolite and subsequent analysis of the *bulk* composition thereof, there may be a significant amount of material such as X₂O₃ which is not an integral part of *the framework* structure of said zeolite but is adsorbed/occluded outside the framework structure.

The XRD pattern of the demetallated SUZ-4 is substantially the same as that shown for the unmodified H-form of the zeolite since the framework of the zeolite remains substantially unchanged except for the loss of some of the X atoms therefrom. There may be some minor modification of the peak intensities as a consequence but the overall XRD pattern would remain the same.

In carrying out the skeletal isomerization process of the present invention, the hydrocarbon feedstock comprising the linear olefins to be isomerized is brought into contact with the H-SUZ-4 zeolite as such or after demetallation, such as eg a dealuminated SUZ-4 zeolite which is an aluminosilicate, at a temperature in the range from 250-550°C, preferably from 300-450°C, eg 430°C.

The isomerization process is suitably carried out at a pressure ranging from subatmospheric to 25 bar absolute, preferably from 1 to 5 bar absolute and specifically eg from 1 to 2 bar absolute. In this type of a reaction system, the partial pressure of the C4 olefin may be in excess of 0.7 bar absolute.

The hydrocarbon feedstock is suitably fed over the zeolite or a demetallated derivative thereof at a weight hourly space velocity, ie the weight of of total olefin feed such as eg butenes per hour divided by the weight of the zeolite, in the range from 5-100, preferably from 25-55 and more specifically from 30-55.

The isomerized product can be recovered from the mixture of reaction products by eg conventional distillation techniques.

The present invention is further illustrated with reference to the following Examples:

### A. Synthesis of SUZ-4 Zeolite:

SUZ-4 zeolite was synthesised by dissolving potassium hydroxide (1.93 g) and sodium aluminate (1.92 g, BDH Grade 40% w/w Al₂O_{3,} 30% w/w Na₂O and the rest water) in distilled water (76.53 g). Triethylammonium hydroxide solution (5.0 g, 40% w/w of triethylammonium hydroxide in water was then added to the sodium aluminate solution followed by the addition of Ludox® AS40 colloidal silica (16.88 g). The resultant gel was stirred for 1 hour and then heated in a revolving autoclave at 180°C for 96 hours. After this period, the product was removed from the autoclave, the solids filtered and washed thoroughly with distilled water. The resultant washed solid was then dried overnight ast 120°C. XRD analysis revealed the solid to be highly crystalline SUZ-4 zeolite. This solid zeolite was then calcined at 550°C in air for 16 hours. Microanalysis revealed the silica : alumina ratio of the product to be 11.2.

The calcined SUZ-4 produced in step (A) was converted to the active acid form by contacting the solid zeolite (5.0 g) with ammonium nitrate solution (125 ml, 1.5 molar) at 80°C for 3 hours. After this period, the solid was filtered and washed thoroughly with distilled water and then dried at 120°C. This washing procedure was repeated twice. The resultant ammonium form of SUZ-4 zeolite was then calcined at 500°C for 2 hours.

### B. Preparation of de-alumination SUZ-4 zeolite in its H-Form:

A SUZ-4 zeolite was synthesised using the following procedure: Potassium hydroxide (4.43 g) was dissolved in distilled water (98.92 g). Fumed silica (14.24 g; CABOSIL®) was then added to this solution with vigorous stirring for a few minutes until homogeneous followed by the addition of triethyl ammonium hydroxide solution (14.54 g, 40% w/w of triethylammonium hydroxide in water) and quinuclidine (4.39 g) as template when a thick gel was formed. Sodium aluminate (2.5 g, same Grade as in (A) above) was dissolved in distilled water (20 g) and the solution was then added to the silica gel (referred to above) with vigorous stirring for 1 hour. The gel was then transferred to an autoclave and heated at 135°C for 166 hours. Thereafter, the reaction mixture was removed, filtered, washed thoroughly with distilled water and then dried at 120°C overnight. XRD analysis of this product revealed the solid to be highly crystalline SUZ-4. This material was then calcined at 550°C for 10 hours.

5.0 g of sample from preparation (C) above was crushed and sieved to a particle size in the range from about 0.5mm to 1.0 mm. The crushed particles were then hydrothermally treated at 560°C in a glass micro-reactor for 150 minutes using distilled water fed at a rate of 10 ml/hour and nitrogen (flow rate = 100 ml/min) as carrier gas. After the hydrothermal treatment, the treated solid particles were acid washed by mixing with hydrochloric acid (50 ml, 1 molar) at room temperature for 2 hours. The acid-washed solid was then filtered and washed with copious amounts of distilled water. Microanalysis of this washed solid revealed a silica to alumina ratio of 13.1 in the dealuminated zeolite.

### EXAMPLE 1:

### Skeletal Isomerization Reaction with De-aluminated SUZ4:

The particulate solid, de-aluminated SUZ-4 zeolite produced in Section (A) above was pressed into tablets under 10 tonnes in a SPECAC® tabletting press. The tablets were then broken and sieved into granules to pass through a 1000µm sieve but not through a 500µm sieve.

A 5.0 ml volume of this de-aluminated, sieved zeolite (1.96 g) was loaded into a 3-Zone Severn Sciences stainless steel reactor tube having an outer diameter of 1.59 cm (0.625 inch) and an internal diameter of 1.27 cm (0.5 inch). The reactor was fitted with concentric thermowell of 0.32 cm (0.125 inch) outer diameter containing a thermocouple to monitor the process temperature. The catalyst was located in the central zone (Zone 2) of the 3-Zone reactor. A preheater consisting of 20 ml of carborundum (medium, about 80 grit) was employed in Zone 1 of the reactor in order to ensure that feed gases were at the desired temperature for catalyst testing.

The catalyst was activated in a nitrogen gas stream (1000 ml/hr feed rate). Under nitrogen flow, the reactor temperature was ramped at 5°C per minute from ambient to the desired initial operating temperature of 420°C. The operating pressure of the reactor was controlled by a back pressure regulator.

The reactant hydrocarbon feed stream consisted of Raffinate II which is a C4 olefin containing stream obtained as by-product from the industrial preparation of polyisobutene having the composition shown in the Tables below.

Raffinate II was stored as liquid in a pressure vessel under top pressure of 8 bar gauge nitrogen. The liquid was pumped through a back pressure regulator to maintain a desired weight hourly space velocity of about 50.1.

Once the desired reactor temperature was attained, the liquid stream was brought on-line to the reactor. The nitrogen flow was then maintained for 15 minutes and then stepped back by 25%. This process continued for one hour at which stage the catalyst was exposed to purely C4 feedstock. This resulted in a 8-10°C exotherm being observed bringing the catalyst bed temperature during running under the olefinic hydrocarbon feed only up to 430°C. The catalyst performance was monitored by collection and analysis of gaseous and liquid products using gas chromatographic procedures previously described. Effluent gas and liquid streams were separated using ice-water chilled separator vessel.

The summary of the results achieved are shown in Table 1 below:

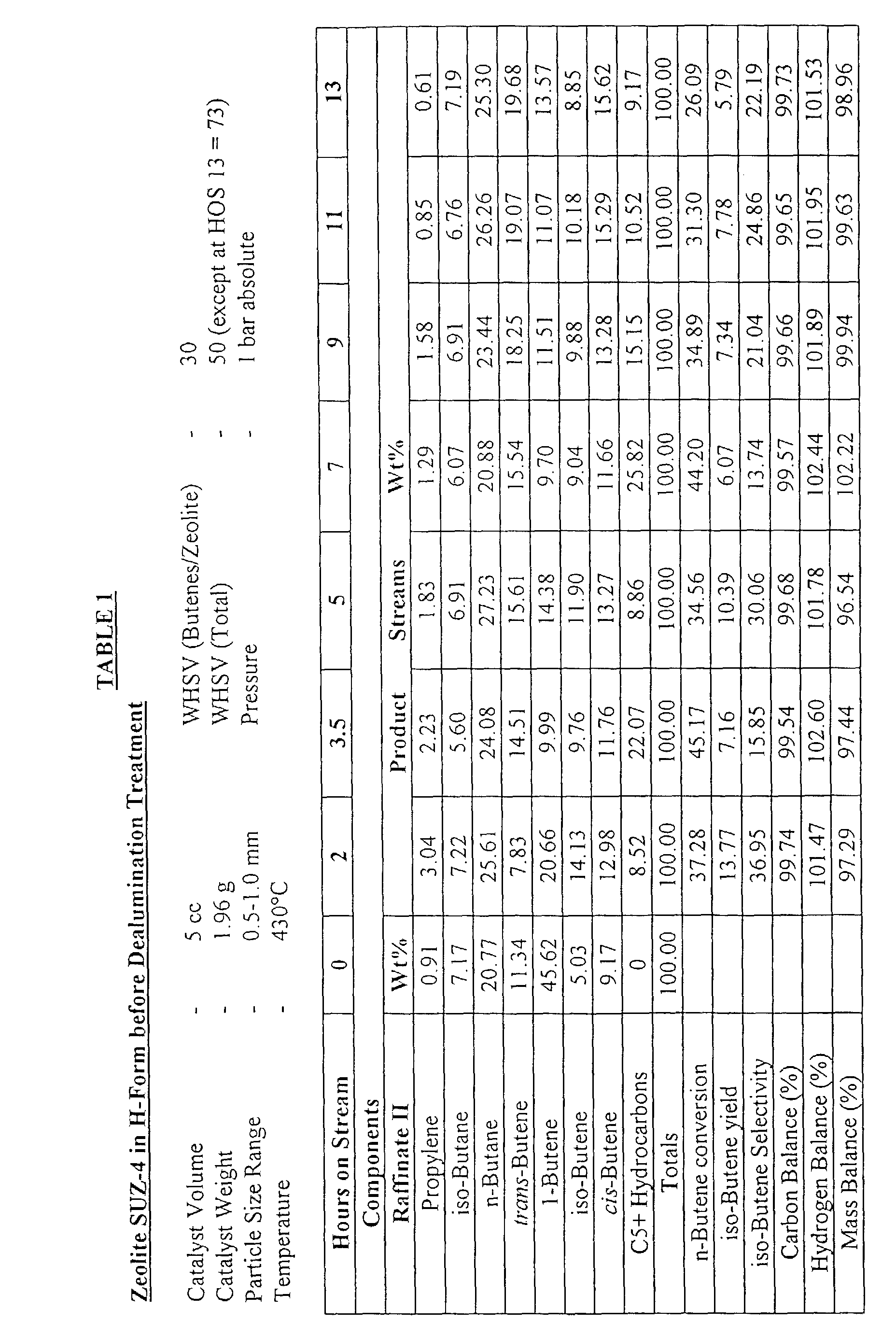

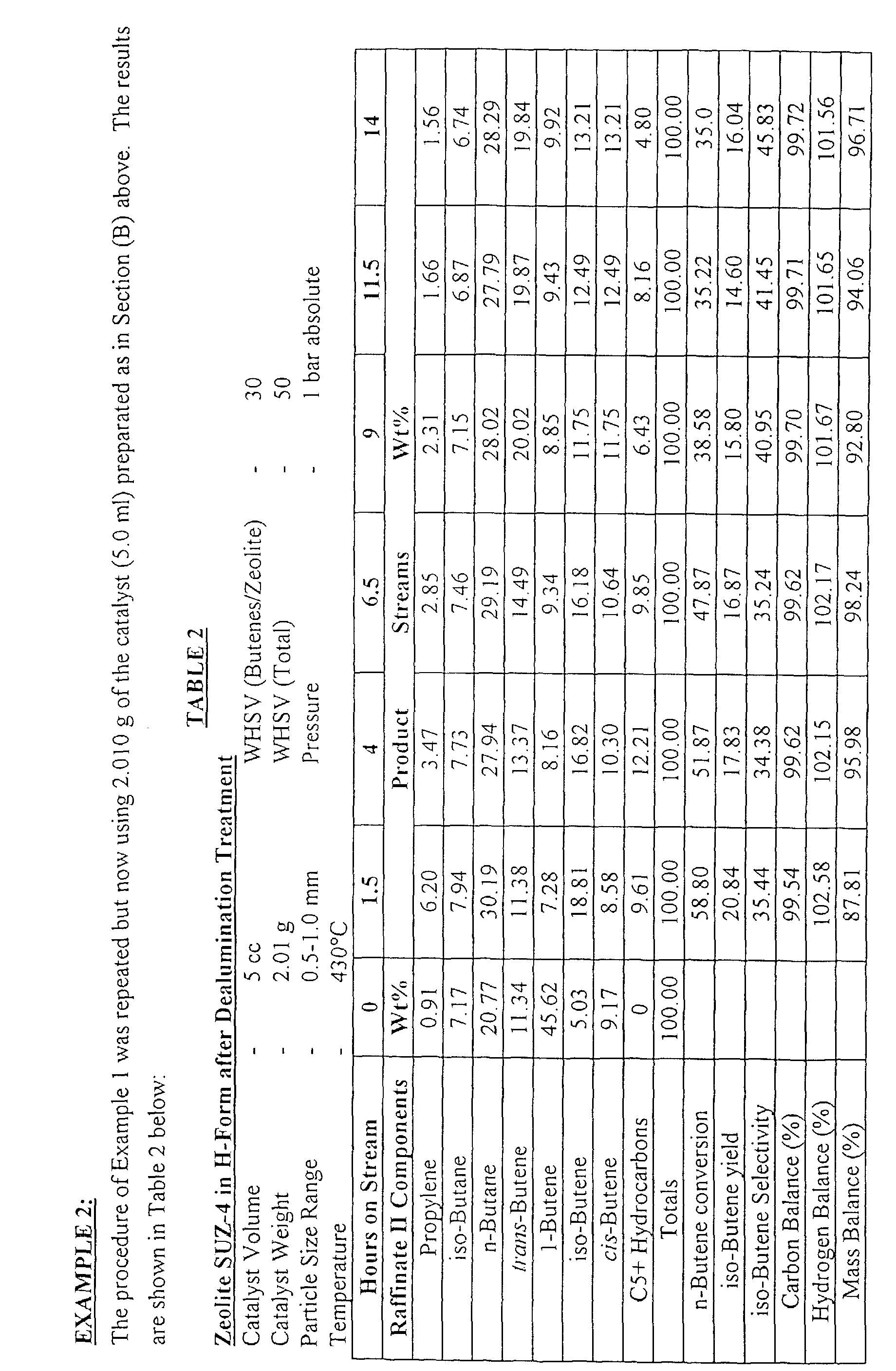

## Claims

1. A process for the skeletal isomerization of hydrocarbon feedstock comprising linear olefins to a product enriched in branched olefins in the presence of a crystalline zeolite, characterised in that the crystalline zeolite is a zeolite SUZ-4 of the empirical formula:
*m*(M_{2/}_{*a*}O):X₂O₃:*y*YO₂ (I)
in which *m* is 0.5 to 1.5; M is a cation of valency *a;* X is a metal ofvalency 3 selected from aluminium, boron, gallium and iron; Y is silicon or germanium and *y* is at least 5; and has in its calcined hydrogen form, an x-ray diffraction pattern including significant peaks substantially as shown in Table A hereinafter wherein
d(Å) - d-spacing
I/I_{O} - Relative Intensity of the peak
VS - 60-140
S - 40-60
M - 20-40
W - 0-20
**TABLE A**
| d(Å) | I/I_{O} | d(Å) | I/I_{O} | d(Å) | I/I_{O} |
|---|---|---|---|---|---|
| 11.5 ± 0.25 | VS | 4.00 ± 0.10 | W | 3.49 ± 0.07 | S |
| 7.50 ± 0.15 | M | 3.95 ± 0.08 | M | 3.48 ± 0.07 | M |
| 7.20 ± 0.15 | W | 3.81 ± 0.08 | M | 3.14 ± 0.07 | M |
| 5.88 ± 0.12 | S | 3.75 ± 0.08 | W | 2.97 ± 0.06 | W |
| 5.73 ± 0.12 | M | 3.67 ± 0.08 | W | 2.93 ± 0.06 | M |
| 4.75 ± 0.10 | M | 3.58 ± 0.08 | S | 2.91 ± 0.06 | W |
| 4.58 ± 0.10 | M/S | 3.55 ± 0.08 | S | - | - |

2. A process according to Claim 1 wherein the hydrocarbon feedstock subjected to skeletal isomerization comprises linear olefins having 4-10 carbon atoms.

3. A process according to Claim 1 or 2 wherein the hydrocarbon feedstock is sourced from the (i) by-products of one or more refinery processes selected from steam cracking, fluid catalytic cracking and catalytic cracking, (ii) butadiene raffinates (Raffinate I) which contain significant proportions of C4 olefins, (iii) the unpolymerised by-products recoverable when Raffinate I is polymerised to form polybutenes and (iv) the by-product gases resulting from the production of the lead-free anti-knock compound, methyl tertiary butyl ether.

4. A process according to claim 3 wherein in (iii) the polymerisation is using a Lewis acid catalyst.

5. A process according to any one of the preceding Claims wherein the hydrocarbon feedstock comprises a C4 linear olefin optionally diluted with C3-C12 alkanes, heavy ends and an inert gas such as e.g. nitrogen.

6. A process according to any one of the preceding Claims wherein the hydrocarbon feedstock contains 30-90% w/w of a linear olefin.

7. A process according to any one of the preceding Claims wherein unconverted linear olefins in the products from the isomerization step are recycled back to the isomerization stage after separation of the branched olefins so formed.

8. A process according to any one of the preceding Claims wherein the crystalline zeolite SUZ-4 is an aluminosilicate.

9. A process according to any one of the preceding Claims wherein the skeletal isomerization of the linear olefins in a given hydrocarbon feedstock to the corresponding branched olefins is achieved by using a demetallated hydrogen form of the SUZ-4 zeolite.

10. A process according to Claim 9 wherein SUZ-4 zeolite in its H-Form is subjected to demetallation by a method selected from:
a. the direct synthetic route in which a portion of the X atoms is replaced with a less acidic atom; and
b. a post-synthetic treatment selected from steaming, acid treatment and reaction with a compound of Y.

11. A process according to any one of the preceding Claims wherein the hydrocarbon feedstock comprising the linear olefins to be isomerized is brought into contact with the H-SUZ-4 zeolite at a temperature in the range from 250-550°C.

12. A process according to any one of the preceding Claims wherein the isomerization process is carried out at a pressure ranging from subatmospheric to 25 bar absolute.

13. A process according to Claim 12 wherein the partial pressure of the C4 olefin is in excess of 0.7 bar absolute.

14. A process according to any one ofthe preceding Claims wherein the hydrocarbon feedstock is fed over the zeolite at a weight hourly space velocity in the range from 5-100.

## Patentansprüche

1. Verfahren zur Gerüstisomerisierung von lineare Olefine umfassender Kohlenwasserstoff-Beschickung zu einem Produkt, das mit verzweigten Olefinen angereichert ist, in Gegenwart eines kristallinen Zeoliths, dadurch gekennzeichnet, daß der kristalline Zeolith ein Zeolith SUZ-4 der empirischen Formel ist:
m(M_{2/a}O):X₂O₃:yYO₂ (I)
worin m 0,5 bis 1,5 ist; M ein Kation der Wertigkeit a darstellt; X ein Metall der Wertigkeit 3 darstellt, ausgewählt aus Aluminium, Bor, Gallium und Eisen; Y Silizium oder Germanium darstellt und y mindestens 5 ist; und in seiner calcinierten Wasserstofform ein Röntgenbeugungsmuster aufweist, das signifikante Peaks einschließt, die im wesentlichen in Tabelle 1 gezeigt werden:
**TABELLE 1**
| d(Å) | I/I_{O} | d(Å) | I/I_{O} | d(Å) | I/I_{O} |
|---|---|---|---|---|---|
| 11,5±0,25 | VS | 4,00±0,10 | W | 3,49±0,07 | S |
| 7,50±0,15 | M | 3,95±0,08 | M | 3,48±0,07 | M |
| 7,20±0,15 | W | 3,81±0,08 | M | 3,14±0,07 | M |
| 5,88±0,12 | S | 3,75±0,08 | W | 2,97±0,06 | W |
| 5,73±0,12 | M | 3,67±0,08 | W | 2,93±0,06 | M |
| 4,75±0,10 | M | 3,58±0,08 | S | 2,91±0,06 | W |
| 4,58±0,10 | M/S | 3,55±0,08 | S | - | - |

2. Verfahren nach Anspruch 1, wobei die Gerüstisomerisierung unterzogene Kohlenwasserstoff-Beschickung lineare Olefine mit 4-10 Kohlenstoffatomen umfaßt.

3. Verfahren nach Anspruch 1 oder 2, wobei die Kohlenwasserstoff-Beschickung von (i) den Nebenprodukten von einem oder mehreren Raffinerieverfahren, ausgewählt aus Dampfcracken, FCC (fluid catalytic cracking) und katalytischem Cracken, (ii) Butadien-Raffinaten (Raffinat I), die wesentliche Anteile an C₄-Olefinen enthalten, (iii) den unpolymerisierten Nebenprodukten, die gewinnbar sind, wenn Raffinat I beispielsweise unter Verwendung eines Lewis-Säure-Katalysators zu Polybutenen polymerisiert wird und (iv) den Nebenproduktgasen, die bei der Herstellung der bleifreien Anti-Klopf-Verbindung Methyltertiärbutylether entstehen, stammt.

4. Verfahren nach einem der vorangehenden Ansprüche, wobei die Kohlenwasserstoff-Beschickung ein lineares C₄-Olefin, gegebenenfalls verdünnt mit C₃-C₁₂-Alkanen, Nachlauf und Inertgas, wie beispielsweise Stickstoff, umfaßt.

5. Verfahren nach einem der vorangehenden Ansprüche, wobei die Kohlenwasserstoff-Beschickung 30-90% Gewicht/Gewicht eines linearen Olefins enthält.

6. Verfahren nach einem der vorangehenden Ansprüche, wobei nicht umgesetzte lineare Olefine in den Produkten aus dem Isomerisierungsschritt nach Abtrennung der so gebildeten verzweigten Olefine zu der Isomerisierungsstufe zurückgeführt werden.

7. Verfahren nach einem der vorangehenden Ansprüche, wobei die Gerüststruktur des kristallinen Zeoliths SUZ-4 in seiner calcinierten Wasserstofform durch das in Tabelle 1 gezeigte Röntgenbeugungsmuster davon charakterisiert wird, wobei
d(Å) - d-Abstand
I/I_{O} - relative Intensität des Peaks
VS - 60-140
S - 40-60
M - 20-40
W - 0-20
**TABELLE 1**
| d(Å) | I/I_{O} | d(Å) | I/I_{O} | d(Å) | I/I_{O} |
|---|---|---|---|---|---|
| 11,5±0,25 | VS | 4,00±0,10 | W | 3,49±0,07 | S |
| 7,50±0,15 | M | 3,95±0,08 | M | 3,48±0,07 | M |
| 7,20±0,15 | W | 3,81±0,08 | M | 3,14±0,07 | M |
| 5,88±0,12 | S | 3,75±0,08 | W | 2,97±0,06 | W |
| 5,73±0,12 | M | 3,67±0,08 | W | 2,93±0,06 | M |
| 4,75±0,10 | M | 3,58±0,08 | S | 2,91±0,06 | W |
| 4,58±0,10 | M/S | 3,55±0,08 | S | - | - |

8. Verfahren nach einem der vorangehenden Ansprüche, wobei der kristalline Zeolith SUZ-4 ein Aluminosilikat ist.

9. Verfahren nach einem der vorangehenden Ansprüche, wobei die Gerüstisomerisierung der linearen Olefine in einer gegebenen Kohlenwasserstoff-Beschickung zu den entsprechenden verzweigten Olefinen unter Verwendung einer entmetallisierten Wasserstofform des Zeoliths SUZ-4 erreicht wird.

10. Verfahren nach Anspruch 9, wobei Zeolith SUZ-4 in seiner H-Form durch ein Verfahren, ausgewählt aus:
a. dem direkten Syntheseweg, bei dem ein Teil der X-Atome durch ein weniger saures Atom ersetzt wird; und
b. einer Nachsynthese-Behandlung, ausgewählt aus Dampfbehandlung, Säurebehandlung und Reaktion mit einer Verbindung von Y, entmetallisiert wird.

11. Verfahren nach einem der vorangehenden Ansprüche, wobei die lineare Olefine umfassende zu isomerisierende Kohlenwasserstoff-Beschickung mit dem Zeolith H-SUZ-4 bei einer Temperatur im Bereich von 250-550°C in Kontakt gebracht wird.

12. Verfahren nach einem der vorangehenden Ansprüche, wobei das Isomerisierungsverfahren bei einem Druck im Bereich von unteratmosphärisch bis 25 bar absolut ausgeführt wird.

13. Verfahren nach Anspruch 12, wobei der Partialdruck des C₄-Olefins oberhalb 0,7 bar absolut liegt.

14. Verfahren nach einem der vorangehenden Ansprüche, wobei die Kohlenwasserstoff-Beschickung über den Zeolith bei einer WHSV (Gewicht-Stunde-Raumgeschwindigkeit) im Bereich von 5-100 eingespeist wird.

## Revendications

1. Procédé pour isomériser au niveau du squelette une charge d'hydrocarbures d'alimentation comprenant des oléfines linéaires en un produit enrichi en oléfines ramifiées en présence d'une zéolite cristalline, caractérisé en ce que la zéolite cristalline est une zéolite SUZ-4 répondant à la formule empirique :
m(M_{2/a}O):X₂O₃:yYO₂ (I)
dans laquelle m a une valeur de 0,5 à 1,5 ; M représente un cation de valence a ; X représente un métal de valence 3 choisi entre l'aluminium, le bore, le gallium et le fer ; Y représente le silicium ou le germanium et y est égal à au moins 5 ; et a sous sa forme hydrogénée calcinée un diagramme de diffraction des rayons X comprenant des pics significatifs correspondant essentiellement à ceux présentés sur le tableau I.
d(Å) - distance d
I/I_{O} - intensité relative du pic Très intense (TI) - 60-140
Intense (I) - 40-60
Moyen (M) - 20-40
Faible (F) - 0-20
**TABLEAU 1**
| d(Å) | I/I_{O} | d(Å) | I/I_{O} | d(Å) | I/I_{O} |
|---|---|---|---|---|---|
| 11,5±0,25 | TI | 4,00±0,10 | F | 3,49±0,07 | I |
| 7,50±0,15 | M | 3,95±0,08 | M | 3,48±0,07 | M |
| 7,20±0,15 | F | 3,81±0,08 | M | 3,14±0,07 | M |
| 5,88±0,12 | I | 3,75±0,08 | F | 2,97±0,06 | F |
| 5,73±0,12 | M | 3,67±0,08 | F | 2,93±0,06 | M |
| 4,75±0,10 | M | 3,58±0,08 | I | 2,91±0,06 | F |
| 4,58±0,10 | M/I | 3,55±0,08 | I | - | - |

2. Procédé suivant la revendication 1, dans lequel la charge d'hydrocarbures d'alimentation soumise à l'isomérisation au niveau du squelette comprend des oléfines linéaires ayant 4 à 10 atomes de carbone.

3. Procédé suivant la revendication 1 ou 2, dans lequel la charge d'hydrocarbures d'alimentation est obtenue à partir (i) des sous-produits d'un ou plusieurs procédés de raffinage choisis entre le craquage à la vapeur d'eau, le craquage catalytique fluide et le craquage catalytique, (ii) de raffinats de butadiène (Raffinat I) qui contiennent des proportions importantes d'oléfines en C4, (iii) des sous-produits non polymérisés pouvant être récupérés lorsque le Raffinat I est polymérisé en utilisant, par exemple, un catalyseur du type acide de Lewis pour former des poly-butènes, et (iv) des sous-produits gazeux résultant de la production du composé anti-détonant sans plomb consistant en méthyl-tertiobutyl-éther.

4. Procédé suivant l'une quelconque des revendications précédentes, dans lequel la charge d'hydrocarbures d'alimentation comprend une oléfine linéaire en C4 facultativement diluée avec des alcanes en C3 à C12, des fractions lourdes et un gaz inerte tel que, par exemple, l'azote.

5. Procédé suivant l'une quelconque des revendications précédentes, dans lequel la charge d'hydrocarbures d'alimentation contient 30 à 90 % en poids/poids d'une oléfine linéaire.

6. Procédé suivant l'une quelconque des revendications précédentes, dans lequel les oléfines linéaires non converties présentes dans les produits résultant de l'étape d'isomérisation sont recyclées de nouveau vers l'étage d'isomérisation après la séparation des oléfines ramifiées ainsi formées.

7. Procédé suivant l'une quelconque des revendications précédentes, dans lequel la structure de réseau de la zéolite cristalline SUZ-4 sous sa forme hydrogénée calcinée est caractérisée par son diagramme de DRX présenté sur le tableau 1, sur lequel :
d(Å) - distance d
I/I_{O} - intensité relative du pic
Très intense (TI) - 60-140
Intense (I) - 40-60
Moyen (M) - 20-40
Faible (F) - 0-20
**TABLEAU 1**
| d(Å) | I/I_{O} | d(Å) | I/I_{O} | d(Å) | I/I_{O} |
|---|---|---|---|---|---|
| 11,5±0,25 | TI | 4,00±0,10 | F | 3,49±0,07 | I |
| 7,50±0,15 | M | 3,95±0,08 | M | 3,48±0,07 | M |
| 7,20±0,15 | F | 3,81±0,08 | M | 3,14±0,07 | M |
| 5,88±0,12 | I | 3,75±0,08 | F | 2,97±0,06 | F |
| 5,73±0,12 | M | 3,67±0,08 | F | 2,93±0,06 | M |
| 4,75±0,10 | M | 3,58±0,08 | I | 2,91±0,06 | F |
| 4,58±0,10 | M/I | 3,55±0,08 | I | - | - |

8. Procédé suivant l'une quelconque des revendications précédentes, dans lequel la zéolite cristalline SUZ-4 est un aluminosilicate.

9. Procédé suivant l'une quelconque des revendications précédentes, dans lequel l'isomérisation au niveau du squelette des oléfines linéaires dans une charge d'hydrocarbures d'alimentation donnée en les oléfines ramifiées correspondantes est réalisée en utilisant une forme hydrogénée démétallée de la zéolite SUZ-4.

10. Procédé suivant la revendication 9, dans lequel la zéolite SUZ-4 sous sa forme H est soumise à une démétallation par un procédé choisi entre :
a. la voie de synthèse directe dans laquelle une partie des atomes X sont remplacés par un atome moins acide ; et
b. un traitement après synthèse, choisi entre le traitement à la vapeur d'eau, le traitement avec un acide et une réaction avec un composé de Y.

11. Procédé suivant l'une quelconque des revendications précédentes, dans lequel la charge d'hydrocarbures d'alimentation comprenant les oléfines linéaires à isomériser est mise en contact avec la zéolite H-SUZ-4 à une température comprise dans l'intervalle de 250 à 550°C.

12. Procédé suivant l'une quelconque des revendications précédentes, dans lequel le processus d'isomérisation est mis en oeuvre à une pression comprise dans l'intervalle allant d'une valeur inférieure à la pression atmosphérique à une pression absolue de 25 bars.

13. Procédé suivant la revendication 12, dans lequel la pression partielle de l'oléfine en C4 est supérieure à une pression absolue de 0,7 bar.

14. Procédé suivant l'une quelconque des revendications précédentes, dans lequel la charge d'hydrocarbures d'alimentation est amenée sur la zéolite à une vitesse spatiale horaire pondérale comprise dans l'intervalle de 5 à 100.
